(19) Europäisches Patentamt
European Patent Office
Office européen des brevets

(11) EP 0 669 938 B1

# (12) FASCICULE DE BREVET EUROPEEN

(45) Date de publication et mention de la délivrance du brevet:
**14.04.1999 Bulletin 1999/15**

(21) Numéro de dépôt: **94928428.5**

(22) Date de dépôt: **22.09.1994**

(51) Int Cl.[6]: **C07K 7/06**, A61K 7/48, A61K 38/07, A61K 38/08, A61K 38/34

(86) Numéro de dépôt international:
**PCT/FR94/01108**

(87) Numéro de publication internationale:
**WO 95/08564 (30.03.1995 Gazette 1995/14)**

(54) **DERIVES PEPTIDIQUES DE L'$g(a)-MSH ET LEUR APPLICATION**

PEPTIDDERIVATE VON ALPHA-MSH UND DEREN ANWENDUNG

PEPTIDE DERIVATIVES OF $g(a)-MSH AND THEIR APPLICATION

(84) Etats contractants désignés:
**AT BE CH DE DK ES FR GB GR IE IT LI LU MC NL PT SE**

(30) Priorité: **22.09.1993 FR 9311281**

(43) Date de publication de la demande:
**06.09.1995 Bulletin 1995/36**

(73) Titulaire: **INSTITUT EUROPEEN DE BIOLOGIE CELLULAIRE**
**F-31520 Ramonville-Saint-Agne (FR)**

(72) Inventeurs:
- **DUSSOURD d'HINTERLAND, Lucien**
  **F-31400 Toulouse (FR)**
- **PINEL, Anne-Marie, Rés. Herbemont,**
  **Le Galoubet**
  **F-31400 Toulouse (FR)**

(74) Mandataire: **Warcoin, Jacques**
**Cabinet Régimbeau,**
**26, avenue Kléber**
**75116 Paris (FR)**

(56) Documents cités:
**EP-A- 0 292 291**

- **JOURNAL OF IMMUNOLOGY, vol. 148, no. 2, 15 janvier 1992, Baltimore, MD (US); N. TUAILLON et al., pp. 445-450**
- **CHEMICAL ABSTRACTS, vol. 108, no. 4, 25 janvier 1988, Columbus, OH (US); K. HASUNUMA, p. 321, AN 26828z**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 669 938 B1

## Description

La présente invention a notamment des dérivés peptidiques de l'α-MSH ("Melanocyte Stimulating Hormone"), présentés sous forme Lipoyl-Peptides.

L'α -MSH et ses homologues ont fait l'objet de nombreuses publications, voire même d'essais thérapeutiques sans que cela se traduise par la réalisation d'un médicament.

La raison principale, est l'extrême ubiquité de 1' α-MSH en fonction des doses administrées et des voies d'administration.

En plus l'absence de relation doses/effets, (ce qui est le cas de nombreuses hormones peptidiques), complique sérieusement son utilisation thérapeutique.

Les récepteurs cellulaires de l' α-MSH et de ses homologues sont du type G et la transduction intra-cellulaire s'effectue selon le cycle de l'AMP cyclique. L'activation de ces récepteurs cellulaires est sensiblement inversement proportionnelle aux doses d'hormones peptidiques, acceptées par les structures du récepteur membranaire.

La présente invention respose sur la recherche de structures peptidiques spécifiquement orientées vers les activités anti-allergiques et anti-inflammatoires d'une part, et activatrices de la mélanogénèse d'autre part à l'exclusion de tout autre effet pharmacologique notamment au niveau du système nerveux central, comme du système nerveux périphérique.

Plus particulièrement, la présente invention concerne un composé comportant une séquence peptidique comprenant au moins une séquence de 4 acides aminés provenant de l'α-MSH, les acides aminés étant sous forme naturelle ou non, ladite séquence étant conjuguée avec l'acide thioctique ou un dérivé de cet acide, sous forme des sels, esters ou amides correspondants.

La séquence de l'α-MSH est la suivante :

N-acétyl - Ser - Tyr - Ser - Met - Glu - His -
Phe - Arg - Trp - Gly - Lys - Pro - Val - $NH_2$ -

Dans cette définition, comme dans ce qui va suivre, les acides aminés peuvent être sous forme D, L ou D,L et les formes non naturelles des acides aminés correspondent à des dérivés, notamment substitués.

L'acide thioctique ou acide α-lipoïque peut se présenter sous la forme oxydée :

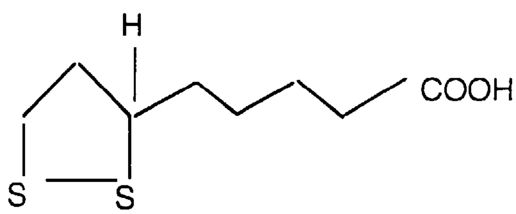

ou sous forme de dérivé dihydrolipoïque :

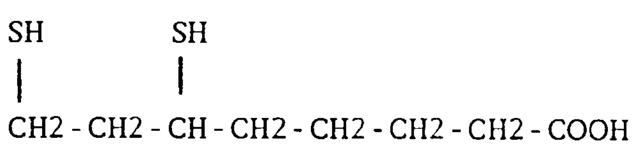

Parmi les dérivés de cet acide, il faut citer le dérivé N-lysine de la forme oxydée ou dihydro.

Un des objets principal de la présente invention, est l'application thérapeutique par voie topique (cutanée) des dérivés précédents.

Ces composés sont des molécules capables de franchir la barrière cutanée, et de présenter la fraction peptidique aux récepteurs cellulaires, en induisant une réponse biologique du type relation doses/effets.

Ces peptides de bas poids moléculaire, dont les séquences d'amino-acides ont été modifiées, sont liés sous forme de sels, d'Esters ou d'amides à des groupements biochimiques actifs, jouant un rôle capital dans le cycle Tricarboxylique (au niveau des Mitochondries notamment). Ces cofacteurs sont les acides lipoïques ou thioctiques sous

forme oxydée ou réduite et leurs dérivés Lipoyl-Lysine, qui sont naturellement liés par covalence aux chaînes polypeptidiques du système enzymatique cellulaire.

Plus spécifiquement, la présente invention concerne des peptides de 4 à 6 acides aminés liés sous forme de Lipoyl-Peptides et de Lipoyl-Lysyl-Peptides à activité anti-allergique, anti-inflammatoire et activateur de la mélanogénèse.

Les composés selon l'invention présentent de préférence la séquence peptidique comportant au moins la séquence suivante :

- His - Phe - Arg -

dans laquelle Phe représente la phénylalanine ou un dérivé halogéné de la phénylalanine, les acides aminés pouvant être sous forme D, L ou D,L, et en particulier il peut s'agir de composés possédant la formule :

[Lip] X - His - Phe - Arg - Y

dans laquelle

Lip est l'acide thioctique ou un de ses dérivés,
X est Glu, OH ou NH2,
Y est

Trp - Gly - OH,
Trp - Gly - NH2
Trp - NH2
Trp - OH

Phe est homo Phe ou p-fluoro Phe,

les acides aminés étant sous forme D, L ou D,L.

L'invention concerne tout particulièrement les composés suivants :

I [(DL) Lip] Glu --- His --- D.homoPhe --- Arg --- Trp --- Gly --- NH2
II [(DH Lip] Glu --- His --- D.homoPhe --- Arg --- Trp --- Gly --- NH2
III [(DL) Lip] Glu --- His --- ParaFluoroPhe --- Arg --- Trp --- Gly --- NH2
IV [(DL) Lip] His --- D.homoPhe --- Arg --- Trp --- NH2
V [N.Lipoyl-Lysine] Glu --- His -- D.homoPhe -- Arg -- Trp --- Gly -- NH2
VI [N.lipoyl-Lysine] His --- D.homoPhe -- Arg -- Trp -- Gly -- NH2
VII [N.lipoyl-Lysine] His --- D.homoPhe --- Arg --- Trp --- NH2

ainsi que les dérivés de ces molécules sous forme de sels d'esters ou d'amides.

Dans les formules, la position de l'acide thioctique correspond au dérivé ester ou amide, la fraction acide de l'acide thioctique assurant la liaison.

Les séquences d'amino-acides mentionnées précédemment peuvent être des séquences d'amino-acides naturels ou des séquences d'amino-acides non naturels. De même dans certains cas il est possible que certains de ces amino-acides comportent des fonctions par exemple ils sont glycosylés et/ou sulfatés.

Il doit être entendu que l'ensemble de ces formes est couvert par la présente description.

La présente invention concerne également des compositions pharmaceutiques ou dermo-cosmétiques comportant un composé tel que défini précédemment.

Les compositions pharmaceutiques peuvent être sous forme administrable par voie orale ou parentérale,

intrapéritonéale ou injectable notamment, mais de préférence sous forme administrable par voie topique externe.

Ces compositions peuvent être, notamment, sous forme de crème, spray ou lotion par exemple et comporter des excipients connus et éventuellement d'autres principes actifs.

Les composés selon la présente invention sont utiles notamment dans la prévention et le traitement des allergies et des inflammations.

Le Peptide III dont la séquence possède en position 3 l'amino-acide paraFluoro Phényl, est particulièrement orienté vers une activité anti-allergique et anti-inflammatoire, par immunosuppression des Monokines (IL1, IL6,$\alpha$ TNF).

Les compositions dermo-cosmétiques sont de préférence sous forme de solution, de lotion, d'émulsion ou de crème utilisables en particulier comme accélérateur de bronzage de la peau sans exposition aux rayons ultraviolets.

Les Peptides II et IV dont les séquences possèdent en position 3 et 2 l'amino-acide D.homo Phényl sont particulièrement orientés vers une stimulation des processus de mélanogénèse et d'activation de la Tyrosinase.

Les séquences Peptidiques selon la présente invention peuvent être obtenues par l'un des procédés quelconques connus de l'homme de métier, notamment par des procédés de synthèse chimique dans laquelle on peut intégrer l'acide thioctique. En tout état de cause compte tenu de la faible dimension des Peptides, la synthèse chimique est tout à fait possible et permet d'obtenir des produits très purs.

**<u>Exemple 1</u>**

**<u>Synthèse du composé 1</u>**

1 - [(DL) Lip] Glu --- His --- D.homoPhe --- Arg --- Trp --- Gly --- NH2

La synthèse est réalisée par la méthode de Merrifield en phase solide en utilisant une résine MBHA et comme groupement protecteur FMOC (fluorényl méthoxy carbonyl).

Les dérivés d' acides aminés utilisés sont :

FMOC ----- Gly ----- OH,
FMOC ----- Arg (Tos) ----- OH,
FMOC ----- Trp ----- OH,
FMOC ----- D.homo.Phe ----- OH,
FMOC ----- His ----- (Trt) ----- OH
FMOC ----- Glu ----- chex) ----- OH

que l'on couple en utilisant le BOP comme agent de couplage.

Chaque acide aminé est utilisé en excés (x2) ainsi que le BOP (x2) et chaque couplage est répété 2 fois.

L'acide Lipoïque est couplé de façon similaire, le groupement FMOC est éliminé à chaque étape par la pipéridine (20 % dans le diméthylformamide).

La déprotection finale est effectuée en deux étapes :

a) acide Trifluoroacétique (2 fois x 5 minutes)
b) acide Fluorhydrique anhydre/p-Crésol 95,5 (45 minutes)

Le composé brut obtenu avec un rendement de 78 % est repris dans un mélange eau/acide acétique 95,5 % et est ensuite lyophylisé.

Le composé 1 obtenu a une pureté de 82 % (HPLC).

100 mg de ce composé sont purifiés par HPLC en utilisant une colonne C18 On obtient 65 mg de produit pur.

Temps de rétention = 17,04 minutes dans les conditions suivantes :

- colonne C8 (250 mm x 5 mm), détection UV 210 nm
- solvant tampon phosphate Triéthylamine - acétonitrile 10-60 % en 15 minutes - débit 1,4 ml /minute.

Analyse des acides aminés :
Glu 1,02 - Gly 1,01 - His 1,00 - Arg 0,94 - D.homo.Phe 1,03 -
**[0040]** Le Tryptophane n'a pas été déterminé car il se dégrade lors de l'hydrolyse acide.
Spectre de masse (FAB +) 1018,4/1126,5

**Exemples 2 et 3**

En remplaçant l'acide DL lipoïque par l'acide DH lipoïque, on obtient le composé 2 :

[DH Lip] Glu -- His --D.homoPhe – Arg -- Trp -- Gly -- $NH_2$.

En remplaçant FMOC D.homoPhe - OH par FMOC ParafluoroPhe - OH, on obtient le composé 3 :

[(DL) Lip] Glu -- His -- ParaFluoroPhe -- Arg - Trp -- Gly -- $NH_2$.

**Exemple 4**

**Synthèse du composé 4**

On opère comme à l'exemple 1 pour préparer :

[(DL) Lip] His ----- D.homoPhe ----- Arg ----- Trp ----- NH2

Constantes physiques...

F - 130°c - Temps de rétention 19,72 minutes
Colonne C18 "Nucléosil" - UV - 279 nm
Solvant TFA - 0,1 % Acétonitrile 75/20
débit - 1,2 ml/minute
Analyse des acides aminés
His = 0,80 - Dh- Phé 1,11 Arg = 0,96
Trp = non dosé - Masse chimique - 887,18

**Exemples 5 à 7**

En opérant comme précédemment, on obtient :

composé 5 [N.Lipoyl-Lysine] Glu -- His - D.homoPhe - Arg - Trp -- Gly - NH2
composé 6 [N.Lipoyl-Lysine] His - D.homoPhe -- Arg - Trp -- Gly -- NH2
composé 7 [N.Lipoyl-Lysine] His -- D.homoPhe -- Arg -- Trp -- NH2

**Exemple 8**

**Etude de l'activité immunosuppressive du dérivé composé 4 sur l'inhibition de la synthèse de l'Interleukine 1 "IL1" chez la souris.**

La sécrétion de l'IL1 est induite par l'administration parentérale de lipopolysaccharides "LPS" à la souris BALB/C.
La libération de lipopolysaccharides bactériens lors des infections microbiennes, induit fortement la synthèse et la libération dans l'organisme des monokines Interleukines "1 IL1" Interleukine 6 "IL6" et du "Tumor - Necrosis - factor" a TNF qui sont reponsables des violentes réactions inflammatoires dont le choc septique.

Matériel

Le composé 4 (P-IV) est mis en solution dans du sérum physiologique. Il est dosé quantitativement par spectrophotométrie à 280 nm (dosage du Tryptophane) ce qui permet un dosage précis du "P-IV" dans les solutions qui sont conservées à -25 ° C.

Lipopolysaccharides "LPS":

Extraits d'Escherichia Coli serotype O127 - B8 - "Réf. SIGMA - L3137" sont utilisés à la concentration de 6 mg/ml dans du sérum physiologique.

Animaux:

Souris BALB/C - femelles - agées de 5 semaines en provenance de IFFA - CREDO soumises à une photopériode de 12 heures de lumière par 24 heures eau et nourriture ad libitum

Méthodes

Deux techniques ont été mises au point pour réaliser ce dosage :

- l'une basée sur un traitement de 5 jours utilisée pour démontrer l'activité anti-inflammatoire du PIV (appelée technique de "SHEEHAN")
- l'autre basée sur un seul traitement, par conséquent beaucoup plus rapide servira pour le contrôle de l'activité du PIV (technique de "DAYNES")

**1- Technique de SHEEHAN**

a) posologie

Trois posologies sont à l'étude :

- 1 ng de PIV dans 0,2 ml de sérum physiologique (soit 5 ng/ml)
- 0,5 ng de PIV dans 0,2 ml de sérum physiologique (soit 2,5 ng/ml)
- 0,1 ng de PIV dans 0,2 ml de sérum physiologique (soit 0,5 ng/ml)

b) Traitement des animaux

Il a été réalisé sur des souris réparties en cinq lots de 5 animaux :

- un lot témoin négatif
- un lot témoin positif
- trois lots PIV (un par posologie)

Les animaux des lots témoins reçoivent une injection de 0,2 ml de sérum physiologique par voie sous cutanée, les animaux des lots PIV reçoivent une injection du produit aux diverses posologies par voie sous cutanée. Ce traitement a lieu pendant 5 jours.

c) Induction de l'IL1 $\alpha$

Quarante huit heures après le traitement par PIV on injecte par voie intrapéritonéale une dose subléthale de LPS (1,2 mg dans 0,2 ml de sérum physiologique) à chaque animal des lots PIV et du lot témoin positif.

d) Obtention des sérums

Cent quatre vingt minutes plus tard toutes les souris sont ponctionnées par les sinus rétroorbitaires sur des tubes secs. Dès que le sang coagule le caillot est décollé. Le sang est centrifugé pendant 10 minutes à 1800 g à la température de 10 ° C.

Les sérums une fois prélevés sont congelés à - 25 ° C

**2- Technique de DAYNES**

a) Posologie

Trois posologies sont étudiées

- 40 µg de PIV dans 0,2 ml de sérum physiologique - soit de 200 µg/ml
- 25 µg de PIV dans 0,2 ml de sérum physiologique - soit de 125 µg/ml
- 10 µg de PIV dans 0,2 ml de sérum physiologique - soit de 50 µg/ml

b) Traitement des animaux

Il a été réalisé sur les souris réparties en 5 lots de 5 animaux

- un lot témoin négatif
- un lot témoin positif
- trois lots PIV (1 par posologie)

Les animaux des lots témoins reçoivent une injection de 0,2 ml de sérum physiologique par voie intraveuneuse.

Les animaux des lots PIV reçoivent une injection du produit aux diverses posologies par voie intraveineuse. (Veine caudale)

c) Induction de l'IL1 α

Immédiatemment après, on injecte par voie intrapéritonéale une dose subléthale de LPS (1,2 mg ans 0,2 ml de sérum physiologique) à chaque animal des lots PIV et aux lots témoins positifs.

d) Obtention des sérums

Cent quatre vingt minutes plus tard toutes les souris sont ponctionnées par les sinus rétroorbitaires sur tubes secs. Dès que le sang coagule le caillot est décollé. Le sang est centrifugé 10 minutes à 1800 g à la température de 10°C. Les sérums une fois prélevés sont congelés à - 25° C.

**3- Dosage de l'IL1 α**

L'Interleukine 1 α contenue dans ces sérums est dosée 24 heures après le prélèvement avec le Kit de dosage ELISA IL1 α murine (réf. 1900 00 GENZYME)

- Description rapide du Kit : la méthode utilisée pour ce dosage est de type sandwich.

On utilise un premier anticorps monoclonal anti-IL1 α murine

On dépose ensuite les échantillons, puis on fait agir un second anticorps anti IL1 α biotinylé.

La révélation a lieu avec de l'avidine couplée à de la peroxydase.

La réaction colorée utilise de la tétraméthylbenzidine (TMB)

La lecture se fait à 450 nm sur un lecteur de plaques MULTISKAN MCC 340 MKII (TITERTEK)

Les résultats obtenus par la méthode de SHEENAN sont rassemblés dans le tableau 1 ci-après :

Tableau 1

| | VALEUR MOYENNE DU TEMOIN POSITIF (en pg/ml) | POURCENTAGE D'ACTIVITE DU PIV PAR POSOLOGIE | | |
|---|---|---|---|---|
| | | 200 µg/ml | 125 µg/ml | 50 µg/ml |
| ETUDE 1 | 250 | -94 % | -50 % | -82 % |
| ETUDE 2 | 230 | -50 % | -46 % | -31 % |
| ETUDE 3 | 200 | -62 % | -37 % | -47 % |
| ETUDE 4 | 430 | -62 % | -49 % | -76 % |
| ETUDE 5 | 430 | -38 % | -62 % | -58 % |

Les diminutions moyennes en IL 1 α obtenues par posologie, sont de l'ordre de 55 % (60% à 200 µg/ml, 50 % à 125 µg/ml et 50 % à 50 µg/ml) pour l'ensemble de cinq études.
L'ensemble des résultats obtenus confirment l'activité anti-inflammatoire de PIV, à travers son action inhibitrice de la synthèse de IL1α.
En effet, avec les deux méthodologies utilisées, on observe une diminution de la synthèse de l'Interleukine 1 α d'au moins 50 % par rapport au témoin.
Cette activité anti-inflammatoire a été complétée d'une étude de l'activité immuno-suppressive du PIV vis à vis du Tumor Necrosis Factor α.
Les résultats obtenus (-40%) confirment l'activité suppressive du PIV sur la synthèse des Interleukines IL 1 et α INF.
On observe également une protection des animaux traités ayant reçu une dose léthale de LPS : cette protection se traduisant par un retard du taux de mortalité hautement significatif.

**Exemple 9**

**Inhibition de l'activité du TNF α par le Peptide PIV**

Matériel et méthode

On utilise les techniques de DAYNES décrites dans l'exemple 8. Technique de DAYNES

a) Posologie
Trois posologies sont à l'étude

- 40 µg de PIV dans 0,2 ml de sérum physiologique - soit de 200 µg/ml
- 25 µg de PIV dans 0,2 ml de sérum physiologique - soit de 125 µg/ml
- 10 µg de PIV dans 0,2 ml de sérum physiologique - soit de 50 µg/ml

b) Traitement des animaux
Il a été réalisé sur les souris réparties en 5 lots de 5 animaux

- un lot témoin négatif
- un lot témoin positif
- trois lots PIV (1 par posologie)

Les animaux des lots témoins reçoivent une injection de 0,2 ml de sérum physiologique par voie intraveuneuse. Les animaux des lots PIV reçoivent une injection du produit aux diverses posologies par voie intraveineuse. (Veine caudale)

c) Induction de TNF α
Immédiatemment après le traitement des souris, on injecte par voie intrapéritonéale une dose subléthale de LPS (1,2 mg dans 0,2 ml de sérum physiologique) à chaque animal des lots et témoin positif.

d) Obtention des sérums
Quatre vingt dix minutes plus tard toutes les souris sont ponctionnées par les sinus rétroorbitaires sur tubes secs. Dès que le sang coagule le caillot est décollé. Le sang est centifugé 10 minutes à 10 ° C à 1800g.
Les sérums une fois prélevés sont congelés à - 25° C.

Dosage du TNFα

Le TNF α contenu dans ces sérums est dosé 24 heures après le prélèvement avec le Kit de dosage ELISA du TNF α murin (réf. 1509-00 GENZYME)

- Description rapide du Kit : la méthode utilisée pour ce dosage est de type sandwich.

On utilise un premier anticorps monoclonal anti-TNF α murin.
On dépose ensuite les échatillons puis on fait agir un second anticorps de chèvre anti TNF α.
La révélation a lieu avec un anticorps d'âne anti-immunoglobuline de chèvre, marqué à la péroxydase.
La réaction colorée utilise l'o-phenylene-diamine (OPD).
La lecture a lieu à 492 nm avec un lecteur de plaques (TITERTEK MKII MCC 340)

Les résultats obtenus sont rassemblés dans le tableau 2 ci-après :

Tableau 2

| | VALEUR MOYENNE DU TEMOIN POSITIF (en pg/ml) | POURCENTAGE D'ACTIVITE DU PIV PAR POSOLOGIE | | |
|---|---|---|---|---|
| | | 200 µg/ml | 125 µg/ml | 50 µg/ml |
| ETUDE 1 | 2600 | -41 % | -26 % | -31 % |
| ETUDE 2 | 4690 | -65 % | -53 % | -38 % |
| ETUDE 3 | 4690 | -52 % | -45 % | -42 % |
| ETUDE 4 | 4040 | -30 % | -41 % | -41 % |
| ETUDE 5 | 4360 | -42 % | -34 % | -31 % |

Cette méthodologie confirme les résultats moyens obtenus précédemment.

En effet, des diminutions moyennes en TNF $\alpha$ obtenues par posologie, sont de l'ordre de 40 % (46 % à 200 µg/ml, 40 % à 125 µg/ml et 37 % à 50 µg/ml) pour l'ensemble des cinq études.

**Exemple 10**

**Etude de l'activité Antiallergique des composés Peptidiques, par le test d'Hypersensibilité de contact au Dinitrofluorobenzène (DNFB).**

Matériel et méthode

Composé 3 "PIII "Lots 1 - 2 - 3
Composé 4 "PIV "Lots 4 - 5 - 6

Des souris femelles C57 BL/6JICO, âgées de 5 semaines sont réparties en dix lots de dix animaux (cinq par cage), avec libre accès à l'eau et à la nourriture, soumises à une photopériode de douze heures de lumière par vingt-quatre heures.

Les animaux des lots I à 6 reçoivent quotidiennement et par application topique sur la peau rasée du dos, les produits à étudier, mis en solution dans du propylène glycol, sous un volume constant de 50 µl et cela pendant cinq jours consécutifs.

Posologie par souris et par jour

PIII - Lots 1 - 2 - 3 - 2,5 µg - 0,5 µg - 0,1 µg - souris/jour
PIV - Lots 4 - 5 - 6 - 2,5 µg - 0,5 µg - 0,1 µg - souris/jour

Les animaux du lot X servent de témoins (ils reçoivent seulement, 50 µl de propylène glycol par application topique pendant cinq jours).

Au cinquième jour, trente minutes après la dernière application toutes les souris sont sensibilisées avec 25 µl de DNFB ( 2.4 - Dinitro - 1 FluoroBenzène (FLUKA CHEMIKA PURUM à 97 % lot n° 33820890) à 2 % dans un mélange 4 pour 1 d'acétone (SDS réf 0510) et de trioléine (FLUKA) appliqué par voie topique sur la région dorsale de peau rasée.

Au sixième jour, on procède à une nouvelle sensibilisation par application de DNFB.

Au onzième jour, l'épaisseur des oreilles des souris, est mesurée à l'aide d'un micromètre (ROCH 0 à 25 mm au 1/100 mm) afin d'obtenir des valeurs de base, puis les oreilles sont stimulées par application topique de 20 µl de DNFB à 0,8 %.

Au douzième jour, l'épaisseur des oreilles est à nouveau mesurée. On établit la valeur moyenne de l'épaississement des oreilles pour les souris des lots qui ont reçu les produits à étudier, ainsi que pour les souris du lot témoin.

Le pourcentage de suppression éventuelle sera exprimé par le calcul suivant : $\frac{T-E}{T} x\ 100$

Les résultats sont rassemblés aux tableaux 3 à 6 suivants :

Tableau 3

Résultats PIII

| LOTS P III | NOMBRE DE SOURIS | SENSIBILISATION | STIMULATION | RESULTATS OREILLE GAUCHE % AUGMENTATION | MOYENNES OREILLE DROITE % AUGMENTATION |
|---|---|---|---|---|---|
| LOT 1 P III (S1) 2,5 µg/souris/jour | 10 | DOS DNFB 2 % | OREILLES DNFB 0,8 % | 19,21 | 19,21 |
| LOT 2 P III (S1) 0,5 µg/souris/jour | 10 | DOS DNFB 2 % | OREILLES DNFB 0,8 % | 19,81 | 19,81 |
| LOT 3 P III (S1) 0,1 µg/souris/jour | 10 | DOS DNFB 2 % | OREILLES DNFB 0,8 % | 17,71 | 17,71 |
| LOT X Témoins | 10 | DOS DNFB 2 % | OREILLES DNFB 0,8 % | 58,92 | 58,92 |

Tableau 4

Résultats PIV

| LOTS PIV | NOMBRE DE SOURIS | SENSIBILISATION | STIMULATION | RESULTATS OREILLE GAUCHE % AUGMENTATION | MOYENNES OREILLE DROITE % AUGMENTATION |
|---|---|---|---|---|---|
| LOT 4 P IV (S2) 2,5 µg/souris/jour | 10 | DOS DNFB 2 % | OREILLES DNFB 0,8 % | 17,99 | 17,99 |
| LOT 5 P IV (S2) 0,5 µg/souris/jour | 10 | DOS DNFB 2 % | OREILLES DNFB 0,8 % | 18,03 | 18,03 |
| LOT 6 P IV (S2) 0,1 µg/souris/jour | 10 | DOS DNFB 2 % | OREILLES DNFB 0,8 % | 20,35 | 20,35 |
| LOT X Témoins | 10 | DOS DNFB 2 % | OREILLES DNFB 0,8 % | 58,92 | 58,92 |

Tableau 5

| Résultats PIII<br>Pourcentage de suppression de l'hypersensibilité de contact "DNFB" | | | | | |
|---|---|---|---|---|---|
| **% de suppression par rapport au Lot Témoin**<br>selon la formule : $\frac{T - E}{T}$ x 100 | | | | | |
| LOT 1 (S1)<br>2,5 µg/souris/jour | | LOT 2 (S1)<br>0,5 µg/souris/jour | | LOT 3 (S1)<br>0,1 µg/souris/jour | |
| OREILLE GAUCHE | OREILLE DROITE | OREILLE GAUCHE | OREILLE DROITE | OREILLE GAUCHE | OREILLE DROITE |
| -67,40 | -67,40 | -66,38 | -66,38 | -69,94 | -69,94 |

Tableau 6

| Résultats PIV<br>Pourcentage de suppression de l'hypersensibilité de contact "DNFB" | | | | | |
|---|---|---|---|---|---|
| **% de suppression par rapport au Lot Témoin**<br>selon la formule : $\frac{T-E}{T}$ x 100 | | | | | |
| LOT 4 (S2)<br>2,5 µg/souris/jour | | LOT 5 (S2)<br>0,5 g/souris/jour | | LOT 6 (S2)<br>0,1 µg/souris/jour | |
| OREILLE GAUCHE | OREILLE DROITE | OREILLE GAUCHE | OREILLE DROITE | OREILLE GAUCHE | OREILLE DROITE |
| -69,47 | -69,47 | -69,40 | -69,40 | -65,46 | -65,46 |

PIII et PIV suppriment de façon hautement significative - "67,90 % et 68,11 %" la réaction d'hypersensibilité cutanée induite par administration de DiNitroFluoroBenzène dans les conditions expérimentales décrites précédemment.

**Exemple 11**

**Etude de l'activité anti-inflammatoire de PIII et PIV**

1) But de l'étude :

Mise en évidence de l'effet inhibiteur des peptides PIII et PIV sur la production par des fibroblastes stimulés par l'IL 1, d'un métabolite de l'acide arachidonique, la PGE2.

2) Méthodes :

2-1 Culture de fibroplastes pulmonaires embryonnaires d'origine humaine :

Nous avons choisi d'utiliser la souche ATCC MRC5 entretenue au laboratoire de façon continue et déjà testée par Cannon et al. (J. Immunol., 1986).

Après un repiquage, les fibroplastes sont cultivés dans des micropuits (plaque de 24 puits de 2 cm2, Falcon). L'innoculum est de 30 000 cellules par puits ; le milieu nutritif utilisé est composé de RPMI 1640 (90 %) et de sérum de veau foetal décomplémenté (SVF, 10 %). Le milieu est changé tous les deux jours jusqu'à confluence des cellules.

2-2 Mise en expérience

Les couches monocellulaires ainsi obtenues sont lavées puis préincubées pendant 24 heures dans du milieu frais ne contenant que 1 % de SVF. Les substances à tester sont ajoutées au milieu à différentes concentrations comprises entre 10-6 et 10-4 M. Après 20 minutes d'incubation en présence de ces composés, l'IL1 recombinante humaine (Tebu, France) à la concentration de 5, 2,5 ou 0,5 ng/ml de milieu est mise en contact avec des cellules pendant 18 heures.

A la fin de cette incubation les surnageants sont prélevés et congelés à -80° C jusqu'à l'analyse. Les couches monocellulaires sont fixées au méthanol.

2-3 Dosage de PGE2

Le dosage RIA est effectué selon la méthode décrite par Dray et al. (Europ. J. Invest. 1975). 3 séries d'expériences en duplicate ont été réalisées pour chaque concentration de produit étudié et pour les contrôles. Les résultats sont exprimés en pg/µg d'ADN. L'effet inhibiteur est exprimé en pourcentage par rapport aux contrôles.

Afin d'éviter toute erreur possible due au comptage en microscopie optique, l'ADN a été dosé par méthode fluorimétrique selon le protocole décrit par Brunk et al. (Analytical Biochem., 1979).

Le tableau 7 ci-après rassemble une 1ère série d'essais sur la souche MRC5

Tableau 7

| Action du PIII | | |
|---|---|---|
| **Echantillons PIII** | **$P_{GE2}$ µg/µg d'ADN** | **% d'inhibition** |
| IL1 : 2,5 ng | 389 ± 60 | |
| + PIII $10^{-4}$ M | 179 ± 50 | 53 |
| + PIII $10^{-5}$ M | 232 ± 45 | 40 |
| + PIII $10^{-6}$ M | 292 ± 68 | 24 |
| + PIII $10^{-7}$ M | 364 ± 68 | 6 |
| + PIII $10^{-8}$ M | 335 ± 50 | 13 |
| + PIII $10^{-9}$ M | 320 ± 43 | 17 |
| IL1 : 0,5 ng | 288 ± 25 | |
| + PIII $10^{-4}$ M | 113 ± 25 | 60 |
| + PIII $10^{-5}$ M | 176 ± 37 | 38 |
| + PIII $10^{-6}$ M | 231 ± 55 | 19 |
| + PIII $10^{-7}$ M | 249 ± 60 | 13 |
| + PIII $10^{-8}$ M | 258 ± 35 | 10 |
| + PIII $10^{-9}$ M | 364 ± 56 | pas d'inhibition |

On observe une bonne action inhibitrice de PIII avec rapport effet dose. Cette action ne dépend pas de la concentration en IL1. On n'observe pas d'action à partir de 10 -7M dans les deux cas.

Tableau 8

| Action de PIV | | |
|---|---|---|
| **Echantillons PIV** | **PGE2 pg/mg d'ADN** | **% d'inhibition** |
| IL1 : 5 ng | 7576 ± 310 | |
| + PIV$10^{-4}$ M | 2964 ± 184 | 60 |
| + PIV$10^{-5}$ M | 4580 ± 230 | 38 |
| + PIV $10^{-6}$ M | 4900 ± 340 | 34 |
| + PIV$10^{-7}$ M | 6150 ± 365 | 17 |
| + PIV $10^{-8}$ M | 6142 ± 255 | 17 |
| + PIV $10^{-9}$ M | 5047 ± 279 | 32 |

PIII et PIV possèdent un pouvoir inhibiteur de l'action de l'IL1 et de la PGE2.
Très significative cette action pourrait être liée à un blocage des récepteurs.

**Exemple 12**

**Etude de l'action de P III et PVI sur la mélanogénèse de la souris après l'application topique sous forme de crème dermique (Technique de Warren).**

Matériel et méthode

1) Matériel

- Composé 3 PIII

[(DL) Lip] - Glu - His - ParaFPhe - Arg - Trp - Gly - NH2

- Composé 6 PIV

[N.Lipoyl - Lysine] - His - D.homoPhe - Arg - Trp - Gly - NH2

- Excipient dermique n° 66
  Lano - vaseline - Ac
  Huile de vaseline Pharmacopée
  Cire d'abeille Ethoxyle
  Lanoline purifiée
  PEG - 200

Formules dermiques

Peptides PIII et PVI incorporés à raison de 5 % dans l'excipient dermique n° 66

Animaux

Souris DBA/2 IFFA - CREDO agées de 5 semaines

2) Méthodes

- On délimite sur chaque animal une tonsure dorsale après épilation
- Les produits à essayer sont appliqués par massage à raison de 2 applications par jour, de 50 µl de chaque préparation pendant 5 jours
- Deux jours après la dernière application, on sacrifie les animaux et on prélève des fractions de 50 mg environ de peau traitée.
- Chaque prélèvement est désséché par lyophilisation et pesé
- Les fragments de peau sont ensuite soumis à une hydrolyse enzymatique par la protéase K pendant 72 heures à 45 ° C (Protéase K Merck - 24.568)
- On ajoute aux hydrolysats obtenus 500 µl de Na2CO3 et 20 µl de H2O2 à 35 %
- On met à incuber 30 minutes à 80 ° C
- Après refroidissement on ajoute à chaque échantillon 200µl de chloroforme / méthanol (2 vol/1 vol)
- On centrifuge à 10 000 g
- On répartit la phase aqueuse (200 µl ) dans les puits d'une microplaque "NUNC"

Dosage de la Mélanine

- Le dosage de la mélanine s'effectue à 405 nm à l'aide d'un Multiskan - Titertek - MCC - 340, comparativement à une gamme étalon de mélanine Sigma M. 8631

Résultats

Ils sont exprimés en pourcentage de mélanine comparativement aux excipients considérés comme témoins

I - Crème P III - 5 10-5 M de PIII pour 50 µl de crème
II - Crème PVI - 5 10-5 M de PVI pour 50 µl de crème
III - Excipient 66 - comme témoin T

Moyenne des résultats sur 15 dosages

Tableau 9

| Echantillons | % de Mélanine en mg |
|---|---|
| I Crème PIII | 4 mg %de mélanine |
| II Crème PVI | 71 m %de mélanine |
| III Excipient 66 - Témoin | 0 mg % de mélanine |

Pourcentage de stimulation

QE = quantité moyenne de mélanine par mg de peau - Echantillon
QT = quantité moyenne de mélanine par mg de peau - Témoin

$$\% \text{ de stimulation} = \frac{QE - QT}{QT} X\ 100$$

La crème dermique contenant PVI administré par voie topique, induit de façon hautement significative la synthèse de la Mélanine au niveau de l'épiderme (+71 %).
Cette activité conforme à l'objet de la présente invention est liée à la structure chimique de PVI qui contient des groupements N.Lipoyl - Lysine et D.homoPhenyl.
La crème dermique dont la formule contient PIII administrée par voie topique, n'a pas d'activité sur la stimulation de la Mélanogénèse Epidermique, cette absence d'activité est liée à la présence dans la structure PIII du groupement "para.FluoroPhenyl" conformément à l'objet de la présente invention.
Ces résultats ont été confirmés par une étude sur la Mélanogénèse selon la technique de "CLOUDMAN" par culture de mélanocytes in vitro, en utilisant les cellules du mélanome de CLOUDMAN chez le rat.
L'ensemble des résultats obtenus sont conformes à l'objet de la présente invention dont l'objectif est la réalisation de dérivés peptidiques actifs par voie Topique et dont les activités anti allergiques et anti inflammatoires peuvent être séparées de l'activité sur la Melanogénèse selon les applications envisagées.

**Exemple 13**

**Etude comparative de l'activité des Lipoyl-Peptides et de leurs structures peptidiques, sur l'hypersensibilité de contact au D.N.F.B.**

Matériel et méthode

La méthode utilisée est celle décrite précédemment dans l'exemple 10.
Hypersensibilité de contact induite chez la souris par la Dinitrofluorobenzène -"D.N.B.F."

**Produits étudiés**

Lot I: (DL) - Lip - Glu - His - D.homoPhe - Arg - Trp - Gly - NH2
Lot II: H - Glu - His - D.homoPhe - Arg - Trp - Gly - NH2
Lot III: Témoin = Acide (DL) Lipoïque

Solvant : le propylène Glycol précédemment employé comme témoin dansl'exemple 10.
Une mesure de l'activité du témoin sera effectuée pour chaque expérimentation.

Expérimentation et posologie

Lot I: Doses utilisées

1 = 1 µg/souris/jour
2=10 µg/souris/jour
3 = 100 µg/souris/jour

Lot II : Doses utilisées

4= 1 ng/souris/jour
5=10 ng/souris/jour
6=100 ng/souris/jour

Témoin : dose utilisée "DL-Lipoïc-Acide" = 10 ng par souris et par jour.
Au cinquième jour, trente minutes après la dernière application toutes les souris sont sensibilisées avec 25 µl de D.N.F.B. (2.4-Dinitro-1 FluoroBenzène (FLUKA CHEMIKA PURUM à 97 % lot n° 33820890) à 2 % dans un mélange 4 pour 1 d'acétone (SDS réf. 05510) et de trioléine (FLUKA) appliqué par voie topique sur la région dorsale de peau rasée.
Au sixième jour, on procède à une nouvelle sensibilisation par application de D.N.F.B.
Au onzième jour, l'épaisseur des oreilles des souris, est mesurée à l'aide d'un micromètre (ROCH 0 à 25 mm au 1/100 mm) afin d'obtenir des valeurs de base, puis les oreilles sont stimulées par application topique de 20 µl de D.N.F.B. à 0,8 %.
Au douzième jour, l'épaisseur des oreilles est à nouveau mesurée. On établit la valeur moyenne de l'épaississement des oreilles pour es souris des lots qui ont reçu les produits à étudier, ainsi que pour les souris du lot témoin. Le pourcentage de suppression éventuelle sera exprimé par le calcul suivant: $\frac{T-E}{T} x\ 100$

Tableau 10

| SOURIS : HYPERSENSIBILITÉ DE CONTACT<br>TABLEAU RECAPITULATIF LOT I | | | | | |
|---|---|---|---|---|---|
| LOTS | NOMBRE DE SOURIS | SENSIBILISATION | STIMULATION | RESULTATS | : MOYENNE |
| | | | | Oreille gauche | Oreille droite |
| | | | | Moyenne % Augmentation | Moyenne % Augmentation |
| Lot I 100 ng/ souris/jour | 10 | Dos DNFB 2 % | Oreilles DNFB 0,8 % | 11,66 | 11,66 |
| Lot I 10 ng/ sourie/jour | 10 | Dos DNFB 2 % | Orellles DNFB 0,8 % | 12, 95 | 13,01 |
| Lot I 1 ng/ souris/jour | 10 | Dos DNFB 2 % | Oreilles DNFB 0,8 % | 13,88 | 13,86 |

Tableau 10 (continued)

| SOURIS : HYPERSENSIBILITÉ DE CONTACT<br>TABLEAU RECAPITULATIF LOT I | | | | | |
|---|---|---|---|---|---|
| LOTS | NOMBRE DE SOURIS | SENSIBILISATION | STIMULATION | RESULTATS | : MOYENNE |
| | | | | Oreille gauche | Oreille droite |
| | | | | Moyenne % Augmentation | Moyenne % Augmentation |
| Témoins | 10 | Dos DNFB 2 % | Oreilles DNFB 0,8 % | 56,34 | 56,75 |

Tableau 11

| SOURIS LOT I<br>HYPERSENSIBILITE DE CONTACT - DNFB | | | | | |
|---|---|---|---|---|---|
| % DE SUPPRESSION PAR RAPPORT AU LOT TEMOIN<br>SELON LA FORMULE : $\frac{T-E}{T}$ x 100 | | | | | |
| Lot I :<br>100 ng/sourls/jour | | Lot I :<br>10 ng/souris/jour | | Lot I :<br>1 ng/souris/jour | |
| Orelile gauche | Oreille droite | Oreille gauche | Oreille droite | Oreille gauche | Oreille droite |
| -79,30 | -79,45 | -77,01 | -76,90 | -75,40 | -75,58 |

Tableau 12

| SOURIS : HYPERSENSIBILITÉ DE CONTACT - DNFB<br>TABLEAU RECAPITULATIF LOT II | | | | | |
|---|---|---|---|---|---|
| LOTS | NOMBRE DE SOURIS | SENSIBILISATION | STIMULATION | RESULTATS | : MOYENNE |
| | | | | Oreille gauche | Oreille droite |
| | | | | Moyenne % Augmentation | Moyenne % Augmentation |
| Lot II : 1 ng/ souris/jour | 10 | Dos DNFB 2 % | Orellles DNFB 0,8 % | 37,07 | 37,07 |
| Lot II : 10 ng/ souris/jour | 10 | Dos DNFB 2 % | Oreilles DNFB 0,8 % | 34,84 | 34,84 |
| Lot II : 100 ng/ souris/jour | 10 | Dos DNFB 2 % | Orellles DNFB 0,8 % | 43,10 | 43,10 |
| LOT TEMOINS | 10 | Dos DNFB 2 % | Oreilles DNFB 0,8 % | 58,92 | 58,92 |

Tableau 13

| SOURIS : HYPERSENSIBILITE DE CONTACT - DNFB | | | | | |
|---|---|---|---|---|---|
| % DE SUPPRESSION PAR RAPPORT AU LOT TEMOIN SELON LA FORMULE : $\frac{T-E}{T} \times 100$ | | | | | |
| Lot II : 1 ng/souris/jour | | Lot II : 10 ng/souris/jour | | Lot II : 100 ng/souris/jour | |
| Oreille gauche | Oreille droite | Oreille gauche | Oreille droite | Oreille gauche | Oreille droite |
| 37,08 | 37,08 | 40,83 | 40,87 | 27,15 | 27,15 |

Résultats

Lot I - Lipoyl-Peptide - I

On observe 79 % de suppression de l'hypersensibilité cutanée à la dose de 100 ng/souris/jour.
On observe également une nette relation doses/effets entre les doses de 1 ng - 10 ng et 100 ng.

Lot II - Peptide seul

On observe 40 % de suppression de l'hypersensibilité cutanée à la dose de 10 ng/souris/jour.
Pour les doses de 1 ng et de 100 ng, la réponse suppressive est de l'ordre de 37,08 et 27,15 %, sans que l'on puisse observer une relation du type doses/effets.

Lot témoin - Acide Lipoïque seul

On n'observe pas d'effet suppressif de l'hypersensibilité cutanée par administration de l'acide Lipoïque, en solution dans le Propylène - Glycol.
Administrés par voie topique et dans les conditions expérimentales décrites , la stucture "Lipoyl Peptide" induit de façon hautement significative la suppression de l'hypersensibilité cutanée chez la souris.

**Exemple 14 Embryo - Toxicologie**

**Analyse qualitative et quantitative des effets de P IV sur la différenciation mélanocytaire en culture in vitro.**

Echantillon à analyser - composé 4 = PIV

Matériel biologique

Cellules embryonnaires isolées en culture in vitro dans un milieu défini, purement salin (conditions standard de culture : 2 ml de milieu/culture).
Le territoire embryonnaire est prélevé (sur l'embryon de Pleurodèle et sur l'embryon d'Axolotl) dès la première étape de la mélanogénèse, immédiatement après l'induction mélanocytaire.
A ce stade précoce du développement embryonnaire, le bourrelet neural, qui vient de s'individualiser, renferme l'ensemble des précurseurs mélanoblastiques. La mise en culture des cellules du bourrelet neural et du mésoderme sous-jacent permet de cocultiver (dans des conditions totalement identiques) les mélanoblastes avec différents autres précurseurs cellulaires appartenant à des catégories variées : neuroblastes myoblastes, cellules épidermiques, fibroplastes, cellules mésenchymateuses.
Ceci permet une étude cinétique comparative, sur le vivant, des effets de PIV sur les mélanocytes et divers autres types cellulaires.
Durée de la culture (culture primaire) : 2 à 3 semaines à 20°C.
La différenciation cellulaire apparait morphologiquement dès le 3ème jour et est quasiment terminée à 8-10 jours de culture.

**Traitements**

1° Essais dose/réponse

Une gamme de concentrations de PIV a été analysée :

0,01 µg/ml ; 0,1 µg/ml ; 1 µg/ml ; et 10 µg/ml
0,01 µg/ml :pas d'effet
1 µg/ml : effet optimal sur la différenciation mélanocytaire (suivant les critères indiqués dans "Résultats")

Sont donc retenues pour ces travaux les 2 doses : 0,1 et 1 µg/ml.

2° Traitements

1 seul traitement lors de l'ensemencement des cellules en culture. Après leur attachement (3 jours à 20°C) le milieu est remplacé par du milieu frais sans le Peptide PIV

1 traitement lors de l'ensemencement et un 2ème traitement lors du changement de milieu.

Résultats

Effets d'ordre qualitatif

1°) Effet stimulant de PIV sur la mélanogénèse
2°) Cet effet ne s'accompagne pas de phénomènes de cytotoxicité.
3°) Les autres catégories cellulaires cultivées ne sont pas touchées, leur morphologie et leur dfférenciation se déroulent normalement, excepté celles des neurones qui présentent des prolongements neuritiques plus courts et en réseau plus dense que chez les témoins. Il est à remarquer que ces neurones sont issus de la crête neurale (bourrelet neural) et sont donc les neurones à l'origine du système nerveux périphérique.
4°) Aucun effet toxique ne se manifeste, l'ensemble des cellules, autres que les mélanoblastes, se différencient et évoluent comme les cellules témoins non traitées.

Analyses quantitatives

L'effet suivant de PIV sur la mélanogénèse se traduit non seulement par une augmentation de la taille des mélanocytes (Cf. planche I et II) mais également :

5°) par une augmentation de leur nombre ;
6°) par une augmentation de la quantité de mélanine biosynthétisée.

A - Effet stimulant de PIV sur la mélanogénèse

1°) Il est d'ores et déjà important de souligner que les effets stimulants décrits ci-après se manifestent de façon analogue après 2 traitements ou un seul taitement (dans ce cas, les cellules sont maintenues en présence de PIV pendant les seuls 2 premiers jours de culture, lorsqu'elles sont encore des mélanoblastes morphologiquement indifférenciés.

Il n'est donc pas nécessaire, pour avoir une stimulation optimale, de maintenir ces cellules en présence de PIV pendant toute la période de leur différenciation.

L'effet stimulant de PIV s'est manifesté de façon tout à fait identique sur les cellules embryonnaires de Pleurodèles comme d'Axolotl.

2°) La différenciation phénotypique des mélanocytes apparait plus rapidement dans les cultures traitées (dès le 2ème jour après l'ensemencement) que dans les cultures témoins.
3°) La stimulation de la mélanogénèse porte sur la taille des mélanocytes qui s'accroît d'un facteur x 4 et même x 5 après 8 jours de culture pour un traitement par 1 µg/ml,
4°) L'effet stimulant de PIV porte également sur la quantité de mélanine biosynthétisée.

Déjà la seule observation, sur le vivant, des mélanocytes traités permet de constater qu'ils sont beaucoup plus sombres, malgré leur plus grande taille, que les mélanocytes témoins.
Nous avons mis au point deux dosages de mélanine :

- par HPLC avec détection électrochimique
- par spectrophotométrie (par adaptation de la méthode de TOMITA et al., 1990) à 470 nm.

La mise au point de ces dosages a été effectuée à partir de solutions-standard de mélanine (Sigma, réf. M 8631) afin de définir la sensibilité comparative et e seuil minimal de détection par l'une et l'autre des méthodes. Le µg est le seul minimal dosable par spectrophotométrie, le ng est le seuil inférieur, dosable par HPLC.
Il est toutefois à remarquer que ces deux techniques, notamment le dosage spectrophotométrique, sont utilisables puisque, pour nos cultures cellulaires, 15 explants embryonnaires associés en culture suffisent pour permettre une quantification (en spectrophotométrie).

Tableau 14

| | Cultures témoins (15 explants) | Cultures traitées (15 explants) | Stimulation |
|---|---|---|---|
| 1ère expérience | 830 ng/explant | 2120 ng/explant | x 2,5 |
| 2ème expérience | 950 ng/explant | 2150 ng/explant | x 2,3 |
| 3ème expérience | 1820 ng/explant | 2710ng/explant | x 1,5 |
| 4ème expérience | 1913 ng/explant | 4782 ng/explant | x 2,5 |

Il est à remarquer que les mélanocytes traités ne présentent aucun signe apparent de cytotoxicité. De plus, ils ont une durée de vie équivalente à celle des mélanocytes témoins in vitro.

B - Effets de piv sur les autres categories cellulaires co-cultivees

Cellules épidermiques ciliées et non ciliées, cellules mésenchymateuses, fibroblastes :

- pas de prolifération cellulaire particulière
- pas d'effet stimulant sur leur dimension, leur étalement...
- pas d'effet cytotoxique
- pas de retard ni d'accélaration dans leur différenciation, ni leur comportement in vitro.

Neuroblastes (origine embryonnaire du SNP) :

- pas d'effet stimulant particulier sur le nombre des neurones, ni sur la taille des corps cellulaires (planche II, A).

Effet sur la morphologie du réseau neuritique:
Dans l'ensemble, le réseau neuritique dans les cultures traitées est constitué de neurites plus courts et plus dispersés que les neurites témoins.
Cette observation devra être analysée plus finement.
L'ensemble des observations et des quantifications ont été réalisées sur plusieurs séries expérimentales afin

de contrôler parfaitement la reproductibilité des phénomènes décrits.

**Exemple 15**

**Effets des Peptides PI et PIV sur les cellules embryonnaires en culture in vitro... "Mélanocytes - Neurones - Cellules Musculaires et Fibroblastes..."**

Matériel et méthodes

Les conditions expérimentales mises en oeuvre sont les suivantes :

- dose utilisée 1 µg/ml (définie par essais dose/réponse)
- 1 seul traitement lors de l'ensemencement des cellules ou plusieurs traitements successifs (renouvellement du milieu chaque 48 heures)
- durée de culture : 6 à 8 jours
- quantification

    1) Comptage des mélanocytes
    2) Dosage de mélanine par HPLC et/ou par spectrophotométrie

- Observation quotidienne des cultures
- Ultrastructure en microscopie électronique à transmission.

Effets stimulants de PI et de PIV sur la mélanogenese

Le traitement par le PI et le PIV des cellules embryonnaires précurseurs des mélanocytes, stimule leur différenciation tant sur un plan qualitatif, que sur un plan quantitatif. Le nombre de mélanocytes croît au minimum d'un facteur 2 par culture.
La quantité de mélanine biosynthétisée par cellule croît également d'un facteur équivalent (1,4 ng/cellule) après huit jours de culture.
Des essais préliminaires d'étude en microscopie confocale avec le système SAMBA 2005 de TITN - ALCATEL montrent qu'il sera possible d'analyser les particules de mélanine (mélanosomes) elles-mêmes.
Les formules peptidiques PI et PIV stimulent la différenciation des mélanoblastes. Il est important de souligner le résultat remarquable suivant : elles ne stimulent pas les cellules embryonnaires précurseurs non encore déterminées dans la voie mélanoblastique. Elles ne sont donc pas des inducteurs et n'ont comme cible que le mélanoblaste : pas d'effet sur les cellules précurseurs ; effet stimulant sur les mélanoblastes déjà induits).

Analyse de la cytotoxicité de PI et PIV

- Neuroblastes
    Les deux formules peptidiques étudiées n'ont aucun effet, toxique ou stimulant, sur les neuroblastes ou les neurones.
    La taille des corps cellulaires, la longueur et le nombre de neurites, l'ultrastructure et enfin la durée de vie des neurones traités sont équivalents à ceux des neurones témoins.
- Cellules gliales (astrocytes)
    Aucun effet toxique n'est observé sur les cellules astrogliales traités par PI et PIV qui se différencient normalement et sans retard par rapport aux astrocytes témoins.
- Myoblastes
    Ici encore aucun effet toxique n'est détecté même à un niveau ultrastructural.
    Les myoblastes s'étalent et se différencient sans retard et sans anomalie par rapport aux myoblastes -contrôle.
- Fibroblastes, mesenchyme
    Des résultats analogues sont obtenus : aucun effet toxique n'est observé.
    L'ensemble des travaux de cytologie et de biochimie sur les effets PI et PIV sur la mélanogénèse d'une part et sur la différenciation de diverses autres catégories cellulaires d'autre part, démontrent que :

    - Le PI et le PIV stimulent la mélanogénèse tant au plan qualitatif qu'au plan quantitatif.

- Le PI et le PIV sont sans effet stimulant sur les autres types cellulaires étudiés : neurones, astrocytes, cellules musculaires, fibroplastes,...
- Le PI et le PIV n'ont pas d'effet cytotoxique sur ces diverses catégories cellulaires qui se différencient et évoluent tout à fait normalement.

## Revendications

1. Composé comportant une séquence peptidique comprenant au moins une séquence de 4 acides aminés provenant de l'α-MSH, les acides aminés étant sous forme naturelle ou non, ladite séquence étant conjuguée avec l'acide thioctique ou un dérivé de cet acide, sous forme de sels, esters ou amides correspondants.

2. Composé selon la revendication 1, caractérisé en ce que la séquence peptidique comporte au moins la séquence suivante :

- His - Phe - Arg -

dans laquelle Phe représente la phénylalanine ou un dérivé halogéné de la phénylalanine, les acides aminés pouvant être sous forme D, L ou DL.

3. Composé selon la revendication 2, caractérisé en ce qu'il présente la formule :

[Lip] X - His - Phe - Arg - Y

dans laquelle

Lip est l'acide thioctique ou un de ses dérivés,
X est Glu, OH ou NH2,
Y est

Trp - Gly - OH,
Trp - Gly - NH2
Trp - NH2
Trp - OH

Phe est homo Phe ou p-fluoro Phe,
les acides aminés étant sous forme D, L ou DL.

4. Composé selon l'une des revendications 1 à 3, caractérisé en ce que les dérivés de l'acide thioctique sont choisis parmi l'acide thioctique, l'acide dihydrolipoïque et la N- lipoyl-lysine.

5. Composé selon l'une des revendications 1 à 4, caractérisé en ce qu'il comporte au moins l'une des séquences suivantes :

I [(DL) Lip] - Glu --- His --- D.homoPhe --- Arg --- Trp --- Gly --- NH2
II [(DH Lip] - Glu --- His --- D.homoPhe --- Arg --- Trp --- Gly --- NH2
III [(DL) Lip] - Glu --- His -- ParaFluoroPhe --- Arg -- Trp -- Gly --- NH2
IV [(DL) Lip] His --- D.homoPhe --- Arg --- Trp --- NH2
V [ N.Lipoyl-Lysine] - Glu -- His -- D.homoPhe -- Arg -- Trp -- Gly -- NH2

VI [ N. Lipoyl-Lysine]- His --- D.homoPhe -- Arg --- Trp -- Gly -- NH2

VII[ N.Lipoyl-Lysine] - His --- D.homoPhe --- Arg --- Trp --- NH2

ainsi que les dérivés de ces molécules sous forme de sels d'esters ou d'amides.

**6.** Composé selon l'une des revendications 1 à 5, caractérisé en ce que certains acides aminés sont glycosylés et/ou sulfatés.

**7.** Composition pharmaceutique comportant à titre de principe actif au moins un composé selon l'une des revendications 1 à 5.

**8.** Composition selon la revendication 7, caractérisée en ce qu'elle est formulée pour être administrée par voie orale ou intrapéritonéale.

**9.** Composition selon la revendication 7, caractérisée en ce qu'elle est formulée pour être administrée par voie topique externe.

**10.** Composition selon l'une des revendications 7 à 9, caractérisée en ce qu'elle est destinée au traitement des allergies et/ou des réactions inflammatoires.

**11.** Composition dermo-cosmétique comportant au moins un composé selon l'une des revendications 1 à 6.

**Patentansprüche**

**1.** Verbindung mit einer Peptidsequenz, enthaltend zumindestens eine Sequenz von 4 Aminosäuren aus α-MSH, wobei die Aminosäuren in natürlicher oder nicht natürlicher Form vorliegen, und wobei die Sequenz der Thioctansäure zugeordnet oder ein Derivat derselben ist, in Form von entsprechenden Salzen, Estern oder Amiden.

**2.** Verbindung nach Anspruch 1, dadurch gekennzeichnet, daß die Peptidsequenz mindestens die folgende Sequenz aufweist:

-His-Phe-Arg-

in der Phe Phenylalanin oder ein halogeniertes Derivat des Phenylalanins darstellt, wobei die Aminosäuren in der D-, L-oder DL-Form vorliegen können.

**3.** Verbindung nach Anspruch 2, dadurch gekennzeichnet, daß diese der vorgenannten Formel entspricht:

[Lip] X-His-Phe-Arg-Y

in der Lip die Thioctansäure oder eines ihrer Derivate, X Glu, OH oder NH2,

Y

Trp-Gly-OH,
Trp-Gly-NH2
Trp-NH2
Trp-OH,

Phe homo-Phe oder p-Flur- Phe, sind, und wobei die Aminosäuren in den Formen D, L, oder DL vorliegen.

4. Verbindung nach einem der vorhergehenden Ansprüche 1 bis 3, daß die Derivate der Thioctansäure die Thioctansäure, Dihydroliponsäure oder N-Lipoyl-lysin sind.

5. Zusammensetzung nach einem der Ansprüche 1 bis 4, dadurch gekennzeichnet, daß sie zumindest eine der folgenden Sequenzen umfaßt:

I [(DL) Lip]-Glu---His---D.homoPhe---Arg---Trp---Gly---NH2
II [(DL) Lip]-Glu---His---D.homoPhe---Arg---Trp---Gly---NH2

III [(DL) Lip]-Glu---His---ParaFluorPhe---Arg---Trp--Gly---NH2
IV [(DL) Lip] His---D.homoPhe---Arg---Trp---NH2
V [N.Lipoyl-Lysin]-Glu--His--D.homoPhe--Arg--Trp--Gly--NH2
VI [N.Lipoyl-Lysin]-His---D.homoPhe--Arg--Trp--Gly--NH2
VII [N.Lipoyl-Lysin]-His---D.homoPhe---Arg--Trp---NH2

ebenso wie die Derivate dieser Moleküle in Form ihrer Salze der Ester oder Amide.

6. Verbindung nach einem der Ansprüche 1 bis 5, dadurch gekennzeichnet, daß bestimmte Aminosäuren glycosyliert und/oder sulfatisiert sind.

7. Pharmazeutische Zusammensetzung, die als aktiven Wirkstoff zumindestens eine der Verbindungen gemäß Ansprüchen 1 bis 5 enthält.

8. Zusammensetzung nach Anspruch 7, dadurch gekennzeichnet, daß sie zur oralen oder intraperitonealen Verabreichung formuliert ist.

9. Zusammensetzung nach Anspruch 7, dadurch gekennzeichnet, daß sie zur externen lokalen Behandlung formuliert ist.

10. Zusammensetzung nach einem der Ansprüche 7 bis 9, dadurch gekennzeichnet, daß sie zur Behandlung von Allergien und/oder Entzündungsreaktionen bestimmt ist.

11. Dermokosmetische Zusammensetzung mit Gehalt an mindestens einer der Verbindungen nach den Ansprüchen 1 bis 6.

**Claims**

1. Compound containing a peptide sequence comprising at least one sequence of 4 amino acids obtained from α-MSH, the amino acids being in natural or non-natural form, the said sequence being conjugated with thioctic acid or a derivative of this acid, in the form of the corresponding salts, esters or amides.

2. Compound according to Claim 1, characterized in that the peptide sequence contains at least the following sequence:

**- His - Phe - Arg -**

in which Phe represents phenylalanine or a halogenated derivative of- phenylalanine, it being possible for the amino acids to be in D, L or DL form.

3. Compound according to Claim 2, characterized in that it has the formula:

[Lip] X - His - Phe - Arg - Y

in which

Lip is thioctic acid or one of its derivatives,
X is Glu, OH or NH2,
Y is

Trp - Gly - OH
Trp - Gly - NH2
Trp - NH2
Trp - OH

Phe is homoPhe or p-fluoroPhe,

the amino acids being in D, L or DL form.

**4.** Compound according to one of Claims 1 to 3, characterized in that the thioctic acid derivatives are chosen from thioctic acid, dihydrolipoic acid and N-lipoyl-lysine.

**5.** Compound according to Claims 1 to 4, characterized in that it contains at least one of the following sequences:

I [(DL) Lip] Glu --- His --- D.homoPhe --- Arg --- Trp --- Gly --- NH2
II [(DH Lip] Glu --- His --- D.homoPhe --- Arg --- Trp --- Gly --- NH2
III [(DL) Lip] Glu --- His --- ParaFluoroPhe --- Arg --- Trp --- Gly --- NH2
IV [(DL) Lip] His --- D.homoPhe --- Arg --- Trp --- NH2
V [N.Lipoyl-Lysine] Glu --- His -- D.homoPhe -- Arg -- Trp --- Gly -- NH2
VI [N.Lipoyl-Lysine] His --- D.homoPhe -- Arg -- Trp -- Gly -- NH2
VII [N.Lipoyl-Lysine] His --- D.homoPhe --- Arg --- Trp --- NH2

as well as the derivatives of these molecules in the form of salts of esters or of amides.

**6.** Compound according to one of Claims 1 to 5, characterized in that some amino acids are glycosylated and/or sulphated.

**7.** Pharmaceutical composition containing as active ingredient at least one compound according to one of Claims 1 to 5.

**8.** Composition according to Claim 7, characterized in that it is formulated so as to be administered orally or intraperitoneally.

**9.** Composition according to Claim 7, characterized in that it is formulated so as to be administered via an external topical route.

**10.** Composition according to one of Claims 7 to 9, characterized in that it is intended for the treatment of allergies and/or of inflammatory reactions.

**11.** Dermo-cosmetic composition containing at least one compound according to one of Claims 1 to 6.